(19) Europäisches Patentamt
European Patent Office
Office européen des brevets

(11) Veröffentlichungsnummer: **0 034 665**
**B1**

# (12) EUROPÄISCHE PATENTSCHRIFT

(45) Veröffentlichungstag der Patentschrift: **28.09.83**

(51) Int. Cl.³: **C 07 D 207/27**

(21) Anmeldenummer: **80200280.8**

(22) Anmeldetag: **27.03.80**

(54) **Verfahren zur Herstellung von substituierten Bis-acylhydraziden.**

(30) Priorität: **23.02.80 DE 3006806**

(43) Veröffentlichungstag der Anmeldung: **02.09.81 Patentblatt 81/35**

(45) Bekanntmachung des Hinweises auf die Patenterteilung: **28.09.83 Patentblatt 83/39**

(84) Benannte Vertragsstaaten: **AT BE CH DE FR GB IT LU NL SE**

(56) Entgegenhaltungen:
**DE-A-2 440 633**
**DE-A-2 745 907**
**Chemical Abstracts, Band 92, Nr. 21, 26. Mai 1980, COLUMBUS, OHIO, USA, Seite 637, Spalte 1, Abstract Nr. 180 999p**

**Die Akte enthält technische Angaben, die nach dem Eingang der Anmeldung eingereicht wurden und die nicht in dieser Patentschrift enthalten sind.**

(73) Patentinhaber: **Rütgerswerke Aktiengesellschaft, Mainzer Landstrasse 217, D-6000 Frankfurt a.Main 1 (DE)**

(72) Erfinder: **Orth, Winfried, Dr., Am Schachtelgraben 28, D-6733 Hassloch/Pfalz (DE)**
Erfinder: **Lange, Fritz Walter, Koenigswieserstrasse 26, D-8035 Gauting (DE)**
Erfinder: **Fickert, Werner, Dr., Stockacher Strasse 14, D-6800 Mannheim-Seckenheim (DE)**

**EP 0 034 665 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von substituierten Bis-acylhydraziden

Die Erfindung betrifft ein Verfahren zur Herstellung von substituierten Bis-acylhydraziden der allgemeinen Formel I

$$\underset{\substack{|\\ R^2}}{A-CH}-(CH_2)_n-CO-NH-NH-CO-(CH_2)_m-\underset{\substack{|\\ R^3}}{CH-B} \qquad (I)$$

worin A und B gleich oder verschieden sind und einen Rest der Formel

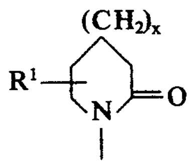

bedeuten, worin $R^1$ Wasserstoff oder eine Alkylgruppe mit 1 bis 4 C-Atomen ist, x den Wert 0 oder 1 hat, und worin $R^2$ und $R^3$ gleich oder verschieden sind und Wasserstoff oder eine Alkylgruppe mit 1 bis 4 C-Atomen bedeuten, n und m gleich oder verschieden sind und 0, 1, 2 oder 3 bedeuten. Die Alkylgruppen können geradkettig oder verzweigt sein.

N,N'-Bisacylhydrazide können durch Umsetzung eines entsprechenden Esters mit einem entsprechenden Säurehydrazid hergestellt werden. Auf diese Weise werden nach der DE-A-2 440 633 oder der deutschen Patentanmeldung DE-A-2 745 907 der F. W. Lange GmbH & Co. KG. Verbindungen der Formel I hergestellt, indem man eine Verbindung der Formel II

$$\underset{\substack{|\\ R^2}}{A-CH}-(CH_2)_n-CO-OR \qquad (II)$$

worin R eine Alkylgruppe mit 1–4 Kohlenstoffatomen und vorzugsweise eine Methylgruppe bedeutet, mit einem Hydrazidderivat der Formel III

$$\underset{\substack{|\\ R^3}}{B-CH}-(CH_2)_m-Co-NH-NH_2 \qquad (III)$$

umsetzt. Selbst bei Verwendung des bevorzugt eingesetzten Methylester ($R=CH_3$) verläuft die Reaktion aber mit einer wirtschaftlich wenig befriedigenden Ausbeute von maximal ca. 50–55%, vgl. zum Verfahren auch die Publikation CA-A-1 065 875.

Aufgabe der Erfindung war es daher, ein Verfahren zur Herstellung von N,N'-Bisacylhydraziden zu finden, das in einfacher Weise mit guten Ausbeuten durchführbar ist, und die Verbindungen I in der gerade für den Einsatz als Pharmazeutika notwendigen hohen Reinheit liefert.

Diese Aufgabe wird durch die vorliegende Erfindung gelöst. Die Erfindung ist dadurch gekennzeichnet, daß man die Umsetzung der Verbindungen der allgemeinen Formel II mit einem Hydrazid der Formel III in Gegenwart eines Amins, quartären Ammoniumhydroxids oder -alkoxids durchführt.

Das erfindungsgemäße Verfahren zur Herstellung der substituierten Bis-acylhydraziden der allgemeinen Formel I

$$\underset{\substack{|\\ R^2}}{A-CH}-(CH_2)_n-Co-NH-NH-CO-(CH_2)_m-\underset{\substack{|\\ R^3}}{CH-B} \qquad (I)$$

worin A und B gleich oder verschieden sind und einen Rest der Formel

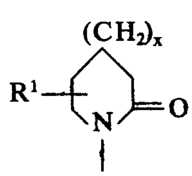

bedeuten, worin $R^1$ Wasserstoff oder eine Alkylgruppe mit 1 bis 4 C-Atomen ist, x den Wert 0 oder 1 hat, und worin $R^2$ und $R^3$ gleich oder verschieden sind und Wasserstoff oder eine Alkylgruppe mit 1 bis 4 C-Atomen bedeuten, n und m gleich oder verschieden sind und 0, 1, 2 oder 3 bedeuten, durch Umsetzung einer Verbindung der Formel II

$$\underset{\displaystyle R^2}{\overset{}{A-\underset{|}{CH}-(CH_2)_n-Co-OCH_3}} \qquad (II)$$

mit einem Hydrazidderivat der Formel III

$$\underset{\displaystyle R^3}{\overset{}{B-\underset{|}{CH}-(CH_2)_m-CO-NH-NH_2}} \qquad (III)$$

worin A, B, $R^1$, $R^2$, $R^3$, x, m und n die oben angegebene Bedeutung haben, ist dadurch gekennzeichnet, daß man die Umsetzung in Gegenwart eines Amins, quartären Ammoniumhydroxids oder -alkoxids durchführt.

Nach diesem Verfahren lassen sich überraschenderweise die Verbindungen der Formel I mit einer wesentlich höheren Ausbeute, die durchschnittlich 70–80% der Theorie beträgt, herstellen.

Die verwendeten Amine sind vorzugsweise primäre, sekundäre oder tertiäre aliphatische, alizyklische oder heterozyklische Amine mit einer oder mehreren, vorzugsweise 1–3 Aminogruppen im Molekül. Außerdem können auch die von diesen Aminen abgeleiteten quartären Ammoniumhydroxide oder auch -alkoxide eingesetzt werden. Als Katalysator kann auch ein Gemisch zweier oder mehrerer Amine verwendet werden.

Im Hinblick auf ihren Einsatz als Pharmazeutika werden für die Herstellung der Verbindungen der allgemeinen Formel I (obwohl für die Umsetzung ohnedies nur geringe Mengen erforderlich sind) solche Amine bevorzugt verwendet, die in bezug auf ihre Toxizität unbedenklich sind. Solche Amine sind z. B. Dimethylaminoäthanol oder Methyldiäthanolamin. Mit diesen Aminen werden außerdem auch sehr gute Ergebnisse erzielt.

Die Verbindungen der Formel II und III werden im allgemeinen im molaren Verhältnis 1 : 1, oder auch mit einem geringen Überschuß (ca. 0,1–0,4 Mol) an II oder III umgesetzt.

Die Menge an zugesetztem Amin ist nicht kritisch und kann z. B. von 0,01 bis 50 Mol-%, bezogen auf die Ausgangssubstanzen II oder III, betragen. Vorzugsweise werden die Amine in Mengen von 0,01–1,0 und insbesondere in Mengen von 0,05 bis 0,2 Mol-% zugesetzt.

Die Umsetzung kann ohne oder insbesondere in Gegenwart eines inerten organischen Lösungsmittels durchgeführt werden. Solche Lösungsmittel sind z. B. niedere Alkanole, wie z. B. Methanol oder Isopropanol, oder aromatische Kohlenwasserstoffe, wie z. B. Toluol oder Xylol, oder auch Gemische davon.

Die Umsetzung wird im allgemeinen bei erhöhter Temperatur, vorzugsweise zwischen 100 und 180° C, und insbesondere zwischen 120 und 140° C durchgeführt, wobei die Wahl der Temperatur auch vom verwendeten Aminkatalysator abhängig ist. Bei Verwendung von quartären Ammoniumverbindungen kann die Reaktionstemperatur unter sonst gleichen Bedingungen im allgemeinen ca. 10–20° tiefer liegen. In bestimmten Fällen kann es auch zweckmäßig sein, das Reaktionsgemisch anfänglich zu kühlen. Die Reaktion kann unter vermindertem Druck, unter erhöhtem Druck im geschlossenen Gefäß, oder insbesondere bei Normaldruck durchgeführt werden. Das Arbeiten unter einer inerten Gasatmosphäre, z. B. mit Stickstoff, kann vorteilhaft sein.

Verbindungen der allgemeinen Formel I besitzen wertvolle pharmakologische Eigenschaften und können z. B. als Psychopharmaka eingesetzt werden. Das erfindungsgemäße Verfahren eignet sich insbesondere zur Herstellung des pharmakologisch hochwirksamen N,N'-Bis(pyrrolidinon-(2)-1-essigsäure)-hydrazids. Die Verbindungen der Formel III können aus den Verbindungen der Formel II und Hydrazin nach dem Verfahren der DE-OS 2 440 633 hergestellt werden.

Bei symmetrischen Bishydraziden kann das erfindungsgemäße Verfahren auch so ausgeführt werden, daß man die Verbindungen der Formel III aus dem bei ihrer Herstellung aus II und Hydrazin erhaltenen Reaktionsgemisch nicht isoliert, sondern zum Reaktionsgemisch direkt die für die weitere Umsetzung erforderliche Menge an Verbindung II zufügt; die Verbindung II kann auch schon vor der Zugabe von Hydrazin in doppelter Menge vorhanden sein. Bei dieser Verfahrensvariante kann das Amin schon der anfänglichen Mischung aus II und Hydrazin zugesetzt werden, vorzugsweise wird es aber dem Reaktionsgemisch erst nach der Umsetzung von II mit Hydrazin zugefügt.

Die vorliegenden Beispiele erläutern das erfindungsgemäße Verfahren näher, ohne es dadurch einzuschränken.

## Beispiel 1

### N,N'-Bis-(pyrrolidinon-(2)-1-essigsäure)-hydrazid

Eine Mischung aus 157 g (1 Mol) Pyrrolidinon-(2)-1-essigsäurehydrazid, 157 g (1 Mol) Pyrrolydinon-(2)-1-essigsäuremethylester, 9 g (0,1 Mol) Dimethylaminoäthanol und 300 ml Xylol wird unter gutem Rühren 24 Stunden auf 130 ± 1° C erhitzt. Das infolge fortschreitender Reaktion freiwerdende Methanol wird über eine kleine Kolonne in dem Maße abdestilliert, daß die eingestellte Sumpftemperatur aufrechterhalten bleibt. Die Reaktionsmasse wird anschließend auf 90° C abgekühlt, 150 ml Isopropanol zugesetzt, das ganze 1 Stunde unter Rühren zum Rückfluß erhitzt, dann erneut auf Raumtemperatur abgekühlt und schließlich abgesaugt, der Filterkuchen mit 3 × 150 ml Isopropanol nachgewaschen und im Vakuum bei 60 – 70° C getrocknet.

Ausbeute: 215 – 19 g (76,2 – 77,5 d. Theorie)
Schmp. 202 – 3° C.

## Beispiel 2

Es wird wie in Beispiel 1 verfahren, jedoch unter Hinweglassen von Dimethylaminoäthanol. Es wird lediglich eine Ausbeute von 50 – 54% d. Theorie erhalten.

In der nachfolgenden Tabelle sind für einige Aminkatalysatoren die nach der Arbeitsweise des Beispiels 1 erhaltenen Ausbeuten zusammengestellt.

| Beispiel Nr. | Katalysator | Einsatz Mol % | Ausbeute % d. Theorie |
|---|---|---|---|
| 3 | 4-Aminopyridin | 0,064 | 74,8 |
| 4 | 1-Benzylimidazol | 0,15 | 72,3 |
| 5 | Benzyl-triäthylammoniumhydroxid | 0,1 | 75,3 |
| 6 | Benzyl-trimethylammoniumhydroxid | 0,1 | 76,0 |
| 7 | 4-Cyclohexylaminopyridin | 0,07 | 73 |
| 8 | Diäthanolamin | 0,12 | 73,1 |
| 9 | Diäthylaminoäthanol | 0,09 | 76,5 |
| 10 | Diäthyl-(2-hydroxy-propylamin) | 0,12 | 72,4 |
| 11 | Diisopropylaminoäthanol | 0,12 | 73,2 |
| 12 | 2-Dimethylaminoäthoxy-pyridin | 0,12 | 73,6 |
| 13 | 4-Dimethylaminopyridin | 0,065 | 75,5 |
| 14 | N,N-Dimethylbenzylamin | 0,14 | 74,8 |
| 15 | Ephedrin | 0,12 | 71,6 |
| 16 | N-2-Hydroxyäthylmorpholin | 0,08 | 75,3 |
| 17 | N-2-Hydroxyähtylpiperazin | 0,065 | 75,4 |
| 18 | N-2-Hydroxyäthylpiperidin | 0,065 | 75,5 |
| 19 | N-2-Hydroxyäthylpyrrolidin | 0,065 | 76,6 |
| 20 | Methyldiäthanolamin | 0,1 | 80,5 |
| 21 | 1-Methylimidazol | 0,14 | 71 |

Fortsetzung

| Beispiel Nr. | Katalysator | Einsatz Mol % | Ausbeute % d. Theorie |
|---|---|---|---|
| 22 | 2-Methylimidazol | 0,14 | 74 |
| 23 | N-Methylmorpholin | 0,15 | 73,1 |
| 24 | 1-Methylpiperazin | 0,1 | 74 |
| 25 | N-Methylpiperidin | 0,1 | 75,4 |
| 26 | N-Methylpyrrolidin | 0,1 | 75,8 |
| 27 | Methyl-cyclohexyl-amin | 0,1 | 73,2 |
| 28 | Morpholin | 0,1 | 72,1 |
| 29 | N,N,N',N'',N''-Pentamethyl-diäthylentriamin | 0,07 | 76,3 |
| 30 | Piperazin | 0,1 | 73,4 |
| 31 | Piperidin | 0,1 | 74,3 |
| 32 | Pyrrolidin | 0,09 | 75,6 |
| 33 | 4-Pyrrolidinopyridin | 0,07 | 76,2 |
| 34 | N,N,N',N'-Tetramethyläthylendiamin | 0,1 | 75,5 |
| 35 | Tetrahydroisochinolin | 0,12 | 72 |
| 36 | Triäthanolamin | 0,092 | 75,5 |

## Beispiel 37

### N,N'-Bis(5-methyl-pyrrolidinon-(2)-1-essigsäure)-hydrazid

Zu einer Mischung aus 171 g 5-Methyl-pyrrolidinon-(2)-1-essigsäurehydrazid und 171 g 5-Methyl-pyrrolidinon-(2)-1-essigsäuremethylester wird eine Lösung von 8 g N-Methylpyrrolidin in 140 ml Xylol gegeben. Diese Mischung wird 24 Stunden unter Rühren auf 130°C erhitzt, wobei das infolge der fortschreitenden Reaktion abgespaltene Methanol über eine Kolonne abdestilliert wird. Nach 5 Stunden Reaktionsdauer tropft man in 1–2 Stunden weitere 60 ml Xylol ein, um die inzwischen auskristallisierende Substanz in Suspension zu halten.

Nach dem Abkühlen auf 80–90°C gibt man 60 ml Isopropanol zu, rührt noch 30 Min. weiter, kühlt dann den Ansatz auf ca. 20°C und saugt schließlich das Kristallisat ab. Den Nutscheninhalt wäscht man noch mit 40 ml Isopropanol nach. Das noch feuchte Produkt wird zu 150 ml Isopropanol in den Kolben zurückgegeben und die Mischung ca. 5 Stunden bei Raumtemperatur gerührt. Dann wird erneut abgesaugt, das Produkt zweimal mit je 40 ml Isopropanol auf der Nutsche nachgewaschen und schließlich bei 70–80°C im Vakuum getrocknet.

Ausbeute: 232 g (75% der Theorie)
Schmp.: 181°C

## Beispiel 38

### N,N'-Bis-(pyrrolidinon-(2)-1-(3-propionsäure)-hydrazid

Eine Mischung aus 171 g Pyrrolidinon-(2)-1-(3-propionsäurehydrazid) und 171 g Pyrrolidinon-(2)-1-(3-propionsäuremethylester) wird mit einer Lösung von 8 g N-Methylpyrrolidin in 140 ml Xylol 24 Stunden unter Rühren auf 130°C erhitzt. Man läßt dann das Gemisch abkühlen, gibt 400 ml Isopropanol zu, erhitzt das Ganze 30 Minuten zum Sieden und läßt schließlich unter Rühren erkalten. Das

Reaktionsprodukt wird abgesaugt, auf der Nutsche mit Isopropanol nachgewaschen und bei 50°C getrocknet.

Ausbeute: 167 g (53,8% der Theorie)
Schmp.: 228°C (unter Zersetzung).

Beispiel 39

N-(Pyrrolidinon-(2)-1-essigsäure)-N'-(5-methyl-pyrrolidinon-(2)-1-essigsäure)-hydrazid

Eine Mischung aus 171 g 5-Methyl-pyrrolidinon-(2)-1-essigsäurehydrazid und 157 g Pyrrolidinon-(2)-1-essigsäuremethylester wird mit einer Lösung von 8 g Dimethylaminoäthanol 24 Stunden unter Rühren auf 130°C erhitzt. Man läßt dann das Reaktionsgemisch abkühlen, gibt 400 ml Isopropanol zu, erhitzt 30 Minuten zum Sieden und läßt schließlich unter Rühren erkalten. Das Reaktionsprodukt wird abgesaugt, auf der Nutsche mit Isopropanol nachgewaschen und bei 50°C getrocknet.

Ausbeute: 157 g (53% d. Theorie)
Schmp.: 159–160°C

**Patentansprüche**

1. Verfahren zur Herstellung von substituierten Bis-acylhydraziden der allgemeinen Formel I

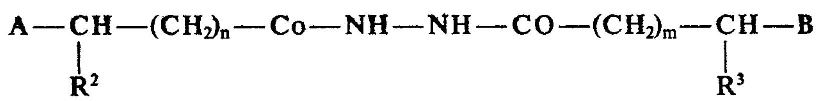

(I)

worin A und B gleich oder verschieden sind und einen Rest der Formel

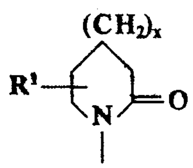

bedeuten, worin $R^1$ Wasserstoff oder eine Alkylgruppe mit 1 bis 4 C-Atomen ist, x den Wert 0 oder 1 hat, und worin $R^2$ und $R^3$ gleich oder verschieden sind und Wasserstoff oder eine Alkylgruppe mit 1 bis 4 C-Atomen bedeuten, n und m gleich oder verschieden sind und 0, 1, 2 oder 3 bedeuten, durch Umsetzung einer Verbindung der Formel II

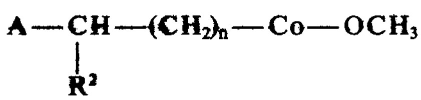

(II)

mit einem Hydrazidderivat der Formel III

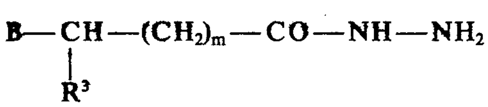

(III)

worin A, B, $R^1$, $R^2$, $R^3$, x, m und n die oben angegebene Bedeutung haben, dadurch gekennzeichnet, daß man die Umsetzung in Gegenwart eines Amins, quartären Ammoniumhydroxids oder -alkoxids durchführt.

2. Verfahren nach Anspruch 1, dadurch gekennzeichnet, daß man das Amin in einer Menge von 0,01 bis 1,0, insbesondere 0,05 bis 0,2 Mol-%, bezogen auf die Verbindungen II oder III, zusetzt.

3. Verfahren nach Anspruch 1 oder 2, dadurch gekennzeichnet, daß man bei einer Temperatur von 100 bis 180°C, insbesondere 120 bis 140°C, arbeitet.

4. Verfahren nach einem der Ansprüche 1 bis 3, dadurch gekennzeichnet, daß man als Amin Dimethylaminoäthanol oder Methyldiäthanolamin einsetzt.

5. Verfahren nach einem der Ansprüche 1 bis 4, dadurch gekennzeichnet, daß man N,N'-Bis-(pyrrolidinon-(2)-1-essigsäure)-hydrazid herstellt.

6. Verfahren nach einem der Ansprüche 1 bis 5, dadurch gekennzeichnet, daß man bei der

Herstellung symmetrischer Bishydrazide der Formel I die Verbindungen der Formel III aus dem bei ihrer Herstellung aus II und Hydrazin erhaltenen Reaktionsgemisch nicht isoliert, sondern zum Reaktionsgemisch direkt die für die weitere Umsetzung erforderliche Menge an Verbindung II zufügt.

## Claims

1. A process for the preparation of substituted Bis-acylhydrazides of the general formula

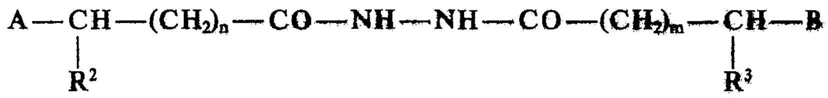

(I)

wherein A and B are identical or different and mean a radical of the formula

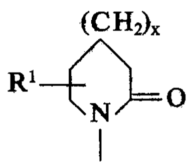

wherein $R^1$ is selected from the group consisting of hydrogen and alkyl of 1 to 4 carbon atoms, x is 0 or 1 and $R^2$ and $R^3$ are individually selected from the group consisting of hydrogen and alkyl of 1 to 4 carbon atoms and n and m are individually 0, 1, 2 or 3 by reacting a compound of the formula

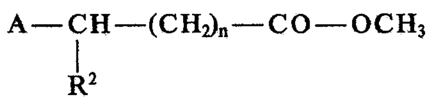

(II)

with a hydrazide of the formula

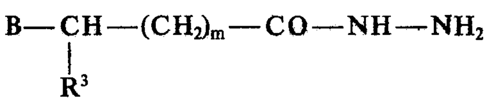

(III)

the improvement comprising effecting the reaction in the presence of an amine, a quarternary ammonium hydroxide or an quarternary ammonium alkoxide.

2. The process of claim 1 wherein the amount of the amine is 0.01 to 1.0, preferred 0.05 to 0.2 mole-percent based on the amount of the compound of formula II or III.

3. The process of claim 1 or 2 wherein the reaction is effected at a temperature of 100 to 180° C, preferred at 120 to 140° C.

4. The process of claims 1 to 3 wherein as amine dimethylaminoethanol or methyl-diethanolamine is used.

5. The process of claims 1 to 4 wherein N,N'-Bis(pyrrolidinone-(2)-1 acetic acid)-hydrazide is prepared.

6. The process of claims 1 to 5 wherein the symetrical bishydrazide of formula III is formed by reacting a compound of formula II with hydrazine and the latter is reacted without isolation with another mole of the compound of formula II.

## Revendications

1. Procédé de préparation de bis-acyl-hydrazines substituées répondant à la formule générale I:

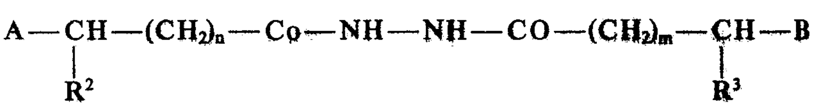

(I)

dans laquelle

A et B sont identiques ou différents et représentent chacun un radical de formule:

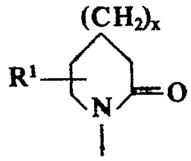

(où $R^1$ représente l'hydrogène ou un radical alkyle contenant de 1 à 4 atomes de carbone, et x est égal à 0 ou à 1),

$R^2$ et $R^3$ sont identiques ou différents et représentent chacun l'hydrogène ou un radical alkyle contenant de 1 à 4 atomes de carbone,

n et m sont identiques ou différents et sont égaux chacun à 0, à 1, à 2 ou à 3,

par réaction d'un composé de formule II:

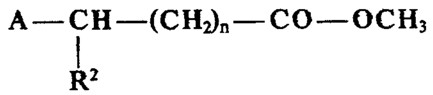

(II)

avec un hydrazide de formule III:

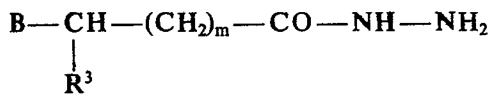

(III)

formules dans lesquelles A, B, $R^1$, $R^2$, $R^3$, x, m et n ont les significations précédemment données, procédé caractérisé en ce qu'on effectue la réaction en présence d'une amine, d'un hydroxyde d'ammonium quaternaire ou d'un alcoolate d'ammonium quaternaire.

2. Procédé selon la revendication 1, caractérisé en ce qu'on ajoute l'amine en une quantité de 0,01 à 1,0 en moles, plus particulièrement de 0,05 à 0,2% en moles, par rapport aux composés II ou III.

3. Procédé selon l'une des revendications 1 et 2, caractérisé en ce qu'on opère à une température de 100 à 180° C, plus particulièrement de 120 à 140° C.

4. Procédé selon l'une quelconque des revendications 1 à 3, caractérisé en ce qu'on utilise, comme amine, le diméthylamino-éthanol ou la méthyl-diéthanolamine.

5. Procédé selon l'une quelconque des revendications 1 à 4, caractérisé en ce qu'on prépare la N,N'-bis-[(oxo-2 pyrrolidinyl-1)-acétyl]-hydrazine.

6. Procédé selon l'une quelconque des revendications 1 à 5, caractérisé en ce que, lors de la préparation de bis-hydrazides symétriques de formule I, on n'isole pas les composés de formule III à partir de mélange réactionnel obtenu lors de leur préparation à partir de II et l'hydrazine, mais on ajoute directement au mélange réactionnel la quantité de composé II nécessaire pour la réaction ultérieure.